(19) Europäisches Patentamt
European Patent Office
Office européen des brevets

(11) **EP 2 590 702 B1**

## (12) EUROPEAN PATENT SPECIFICATION

(45) Date of publication and mention
of the grant of the patent:
**14.05.2014 Bulletin 2014/20**

(21) Application number: **11743866.3**

(22) Date of filing: **04.07.2011**

(51) Int Cl.:
***A61M 16/00*** (2006.01)

(86) International application number:
**PCT/IB2011/052934**

(87) International publication number:
**WO 2012/004718 (12.01.2012 Gazette 2012/02)**

(54) **LEAK ESTIMATION USING LEAK MODEL IDENTIFICATION**

LECKBEURTEILUNG MITTELS LECKMODELLIDENTIFIZIERUNG

ESTIMATION DE FUITE AU MOYEN D'UNE IDENTIFICATION DE MODÈLE DE FUITE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.07.2010 US 362732 P**

(43) Date of publication of application:
**15.05.2013 Bulletin 2013/20**

(73) Proprietor: **Koninklijke Philips N.V.
5656 AE Eindhoven (NL)**

(72) Inventor: **HILL, Peter, Douglas
NL-5656 AE Eindhoven (NL)**

(74) Representative: **van Velzen, Maaike Mathilde
Philips
Intellectual Property & Standards
P.O. Box 220
5600 AE Eindhoven (NL)**

(56) References cited:
**EP-A1- 2 329 768          WO-A1-02/28460
US-A1- 2005 224 078**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to gas delivery systems, such as positive pressure support systems and other ventilator (e.g., invasive) systems, and, more particularly, to a method for estimating leak in a gas delivery system, and a gas delivery system employing such a method.

2. Description of the Related Art

There are numerous situations where it is necessary or desirable to deliver a flow of breathing gas non-invasively to the airway of a patient, i.e., without intubating the patient or surgically inserting a tracheal tube. Such therapies are commonly referred to as non-invasive ventilation (NIV) therapies. For example, it is known to non-invasively deliver continuous positive airway pressure (CPAP) or variable airway pressure, which varies with the patient's respiratory cycle, to treat medical disorders, such as obstructive sleep apnea (OSA), Obesity Hypoventilation Syndrome (OHS) and Chronic Obstructive Pulmonary Disease (COPD).

NIV therapies involve the placement of a patient interface device including a mask component on the face of a patient. The mask component may be, without limitation, a nasal mask that covers the patient's nose, a nasal pillow/cushion having nasal prongs that are received within the patient's nares, a nasal/oral mask that covers the nose and mouth, or a full face mask that covers the patient's face. The patient interface device interfaces the ventilator or pressure support device with the airway of the patient through one or more delivery conduits (together commonly referred to as a patient circuit) so that a flow of breathing gas can be delivered from the pressure/flow generating device to the airway of the patient.

NIV using a single limb patient circuit has safely ventilated patients with respiratory insufficiency for over ten years and those with severe sleep apnea for over twenty years. In NIV, an accurate estimate of the patient flow is required for consistent and accurate volume delivery and for the ventilator to sense the patient's respiratory drive. The accuracy of the estimated patient flow is dependent on three things: (i) the accuracy and precision of the total flow signal (which is measured at the ventilator outlet and which is the composite of the patient flow and the flow caused by leaks (both intentional and unintentional) about the patient interface), (ii) the accuracy and precision of the pressure measurement at the leak sources, and, (iii) the ability to model the leak flow as a function of one or more parameters such as pressure. Thus, one of the key technologies for effective NIV is the estimation of leak flow.

Document US2005/224078 describes a method for treatment of sleep apnea, the method including measuring a total flow of the flow of gas, measuring a pressure of the flow of gas, calculating, based on the total flow and the pressure, a characteristic K of a hypothetical orifice which represents an orifice through which all leakage of gas from the pressure support system occurs, and determining a total instantaneous flow of the leakage of gas from the pressure support system based on the characteristic K of the hypothetical orifice.

Recent clinical practice has begun to increase pressure support in NIV patients. Inspiratory Positive Airway Pressure (IPAP) levels above 30 cmH2O where rarely used ten years ago but now have become common practice for COPD patients. These high pressure support levels amplify errors in the leak estimation. The two primary sources of leak estimation error are (1) noise in the pressure and flow data, and (2) that the assumed leak model is different than that of the actual leak model. In one current leak estimation methodology, known as Auto-Trak™, developed and employed by the assignee of the present invention, the leak model is assumed to be an orifice and the leak is proportional to $P^{½}$, wherein P is the patient pressure. However, the leaks that occur about the mask seals are typically proportional to $P^{\gamma}$, where $\gamma$ is above 1.0. Thus, the actual leak behaves differently than that modeled by $P^{½}$, and the consequence of this leak error is to bias the estimated patient flow negative in the expiratory phase and to bias the estimated patient flow positive in the inspiratory phase. As a result, the triggering and cycling of the positive pressure therapy are desensitized.

The ability to estimate leak flow is also important in situations where it is necessary to deliver a flow of breathing gas to the airway of a patient invasively, i.e., wherein the patient is intubated or has a surgically inserted tracheal tube. Many of the problems with accurately estimating leak flow in NIV systems described above are present in invasive ventilation using a gas delivery system like an invasive ventilator system.

In one embodiment, a method of determining an estimated leak flow $Q_{est}$ in a gas delivery system is provided that includes determining for each of N breaths (N > 1): (i) an average total flow $\overline{Q_{tot}}$ of the gas delivery system, and (ii) M values of $\overline{P^{\gamma}}$ (M > 1) using M $\gamma$ values, wherein each $P$ is a leak pressure of the gas delivery system and each $\gamma$ value is an integer or non-integer real number, determining which one of the $\gamma$ values results in a minimum variation of the values of a coefficient g over the N breaths, wherein for each breath and each $\gamma$ value the coefficient g is determined by $g = \overline{Q_{tot}} / \overline{P^{\gamma}}$, and determining the estimated leak flow $Q_{est}$ using at least the determined one of the $\gamma$ values.

In another embodiment, a gas delivery system is provided that is adapted/programmed to estimate leak flow in the gas delivery system using the method just described.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention. As used in the specification and in the claims, the singular form of "a", "an", and "the" include plural referents unless the context clearly dictates otherwise.

FIG. 1 is a schematic diagram of a pressure support system according to one particular, non-limiting embodiment in which the leak estimation methodology of the present invention may be implemented; and

FIG. 2 is a flowchart showing a methodology for finding g and $\gamma$ for leak estimation according to an exemplary embodiment of the present invention.

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (i.e., a plurality).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

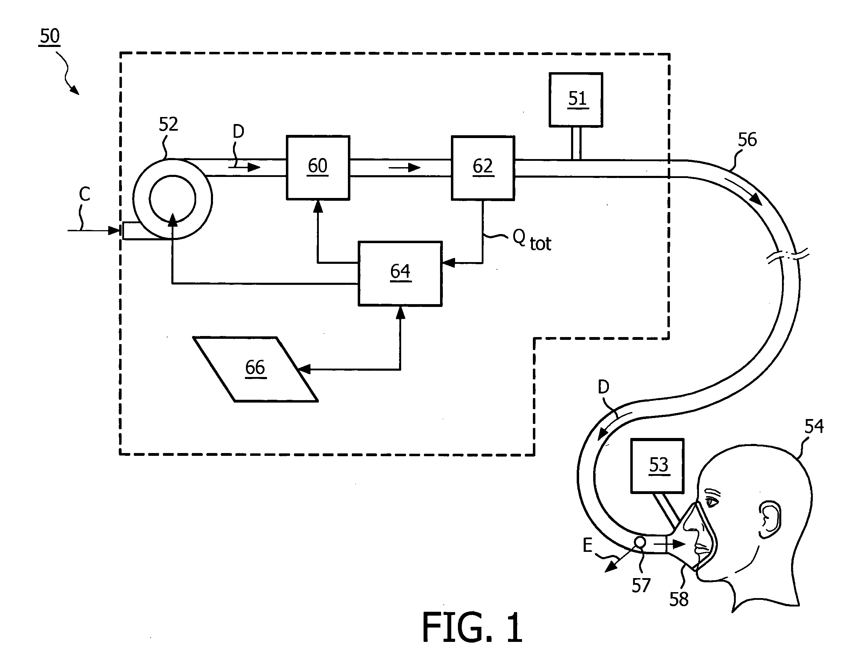

As described in greater detail herein, the present invention solves many of the problems of existing leak estimation methods by providing an improved leak estimation methodology. FIG. 1 is a schematic diagram of pressure support system 50 according to one particular, non-limiting embodiment in which the leak estimation methodology of the present invention may be implemented. It should be understood that pressure support system 50 is meant to be exemplary only for purposes of illustrating and describing the present invention, and that the present invention may be implemented and employed in other types of gas delivery systems, such as, without limitation, a ventilator, such as an invasive ventilator system, that delivers volume controlled ventilation. One such alternative gas delivery system is described in PCT Publication No. WO 2010/044038, entitled "Volume Control in a Medical Ventilator," assigned to the assignee of the present invention. Thus, the present invention may be employed in any type of gas delivery system having leaks where it is necessary or desirable to model leak flow.

Referring to FIG. 1, pressure support system 50 includes gas flow/pressure generator 52, such as a blower used in a conventional CPAP or bi-level pressure support device, piston, bellows, compressor, or any other device that receives breathing gas, generally indicated by arrow C, from any suitable source, e.g., a pressurized tank of oxygen or air, the ambient atmosphere, or a combination thereof. Gas flow/pressure generator 52 generates a flow of breathing gas, such as air, oxygen, or a mixture thereof, for delivery to an airway of a patient 54 at relatively higher and lower pressures, i.e., generally equal to or above ambient atmospheric pressure.

The pressurized flow of breathing gas, generally indicated by arrow D from gas flow/pressure generator 52 is delivered, via a delivery conduit 56, to breathing mask or patient interface 58 of any known construction, which is typically worn by or otherwise attached to patient 54 to communicate the flow of breathing gas to the airway of the patient. Delivery conduit 56 and patient interface device 58 are typically collectively referred to as a patient circuit.

Although not shown in FIG. 1, the present invention also contemplates providing a secondary flow of gas, either alone or in combination with the primary flow of gas (arrow C) from atmosphere. For example, a flow of oxygen from any suitable source, such as an oxygen concentrator, or oxygen storage device (liquid or gas), can be provided upstream of gas flow/pressure generator 52 or downstream of the gas flow generator, for example, in the patient circuit or at the patient interface device, to control the fraction of inspired oxygen delivered to the patient.

Pressure support system 50 shown in FIG. 1 is a single-limb system, meaning that the patient circuit includes only delivery conduit 56 connecting the patient to the pressure support device. As such, exhaust port 57 is provided in the delivery conduit 56 for venting exhaled gasses from the system to atmosphere as indicated by arrow E. It should be noted that exhaust port 57 can be provided at other locations in addition to or instead of in the delivery conduit, such as in the patient interface device 58. It should also be understood that exhaust port 57 can have a wide variety of configurations depending on the desired manner in which gas is to be vented from the pressure support system.

The present invention also contemplates that pressure support system 50 can be a two-limb system, having a delivery conduit and an exhaust conduit connected to patient 54. In a two-limb system (also referred to as a dual-limb system), the exhaust conduit carries exhaust gas from patient 54 and includes an exhaust valve at the end distal from patient 54. The exhaust valve in such an embodiment is typically actively controlled to maintain a desired level or pressure in the system, which is commonly known as positive end expiratory pressure (PEEP).

In the illustrated exemplary embodiment of the present invention, patient interface 58 is a nasal/oral mask. It is to be understood, however, that patient interface 58 can include a nasal mask, nasal pillows, tracheal tube, endotracheal tube, or any other device that provides the gas flow communicating function. Also, for purposes of the present invention, the phrase "patient interface" can include delivery conduit 56 and any other structures that connect the source of pressurized breathing gas to the patient.

It is also to be understood that various components may be provided in or coupled to the patient circuit. For example, a bacteria filter, pressure control valve, flow control valve, sensor, meter, pressure filter, humidifier and/or heater can be provided in or attached to the patient circuit. Likewise, other components, such as muffler and filters can be provided at the inlet of gas flow/pressure generator 52 and at the outlet of valve 60 (described below).

In the illustrated embodiment, pressure support system 50 includes a pressure controller in the form of valve 60 provided in delivery conduit 56. Valve 60 controls the pressure of the flow of breathing gas from gas flow/pressure generator 52 delivered to patient 54. For present purposes, gas flow/pressure generator 52 and valve 60 are collectively referred to as a "pressure generating system" because they act in concert to control the pressure and/or flow of gas delivered to the patient.

It should be apparent that other techniques for controlling the pressure delivered to the patient by the gas flow/pressure generator, such as varying the blower speed, either alone or in combination with a pressure control valve, are contemplated by the present invention. Thus, valve 60 is optional depending on the technique used to control the pressure of the flow of breathing gas delivered to the patient. If valve 60 is eliminated, the pressure generating system corresponds to gas flow/pressure generator 52 alone, and the pressure of gas in the patient circuit is controlled, for example, by controlling the motor speed of the gas flow/pressure generator.

Pressure support system 50 further includes flow sensor 62 that measures the flow of breathing gas within delivery conduit 56 (and thus the flow being output by pressure support system 50). In accordance with the exemplary embodiment shown in FIG. 1, flow sensor 62 is interposed in line with delivery conduit 56, most preferably downstream of valve 60. Flow sensor 62 generates a flow signal $Q_{tot}$ (which, as described elsewhere herein, is the measured total circuit flow) that is provided to controller 64.

Pressure support system 50 further includes one or more pressure sensors that measure the pressure in the patient circuit. As seen in FIG. 1, pressure sensor 51 measures the pressure near the ventilator outlet and pressure sensor 53 measures the pressure on or near patient interface 58. Either or both of these pressure sensors 51, 53 can be used to generate a pressure signal that is representative of the pressure at the source of the leak.

Processing element 64 receives (wired or wirelessly) pressure and flow signals from the pressure and flow sensors (51, 53, 62), respectively, in order to estimate $Q_{leak}$.

Techniques for calculating the patient flow $Q_p$ based on $Q_{tot}$ are well known, and take into consideration the pressure drop of the patient circuit, known leaks from the system, i.e., the intentional exhausting of gas from the circuit as indicated by arrow E in FIG. 1, and unknown leaks from the system, such a leaks at the mask/patient interface. As stated elsewhere herein, the present invention provides an improved methodology for calculating leak flow $Q_{leak}$ (which is described in detail below), which may then be used in calculating $Q_p$ based on $Q_{tot}$.

Controller 64 includes a processing portion which may be, for example, a microprocessor, a microcontroller or some other suitable processing device, and a memory portion that may be internal to the processing portion or operatively coupled to the processing portion and that provides a storage medium for data and software executable by the processing portion for controlling the operation of pressure support system 50, including estimating leak flow $Q_{leak}$ as described in greater detail herein.

Input/output device 66 is provided for setting various parameters used by the variable positive airway pressure support system, as well as for displaying and outputting information and data to a user, such as a clinician or caregiver. It is to be understood that the present invention contemplates providing input/output terminals so that the operation information and data collected by the pressure support system can be monitored and controlled remotely.

Having described an exemplary, non-limiting pressure support system 50 in which the leak estimation methodology of the present invention may be implemented, the leak estimation methodology of the present invention will now be described in detail, including an exemplary, non-limiting embodiment thereof. In addition, for illustrative purposes, the leak estimation methodology of the present invention will be described as being implemented in pressure support system 50. However, it will be understood that the leak estimation methodology of the present invention may be implemented in respiratory positive pressure support systems having different configurations.

$Q_{leak}$ is a composite flow of the intentional leak such as that implemented on patient interface 58 and unintentional leaks that are usually associated with leaks around the seals between the patient interface 58 and the face of patient 54. From the conservation of mass, the following relates total flow from pressure generating system 50, $Q_{tot}$, to the patient flow, $Q_p$ and the leak flow, $Q_{leak}$:

$$Q_{tot}(t) = Q_p(t) + Q_{leak}(t).$$

Thus, $Q_p$ and $Q_{leak}$ are combined to form $Q_{tot}$, the total flow leaving the ventilator, which in the exemplary embodiment is pressure generating system 50.

$Q_{leak}$ is a complex function of pressure at the leak, $P$, and perhaps secondarily, $Q_{tot}$, $Q_p$, patient volume (V) and other measures, either directly or estimated that affect the leak geometry or behavior (indicated generally by X). That function, F, may be expressed as follows:

$$Q_{leak} = F(P, Q_p, Q_{tot}, V, X)$$

One simplification of the leak model uses a single coefficient (G) in front of P raised to an exponent ($\lambda$), and may be expressed as follows:

$$Q_{leak} = GP^{\lambda}.$$

Furthermore, $Q_{leak}$ can be divided into intentional leak, $Q_{il}$, and unintentional leak, $Q_{ul}$, and thus $Q_{leak}$ may be expressed as follows:

$$Qleak = Qil + Qul.$$

The intentional leak component of Qleak is often an orifice and has the general form of

$$Q_{il} = G_{il}P^{\lambda\_il},$$

where $\lambda\_il$ is a complex function of the orifice geometry. Most intentional mask leaks have a $\lambda\_il$ of 0.5 to 0.7. However, the Plateau Exhalation Valve (PEV) has a constant leak flow with respect to pressure and, thus, its gamma is 0. Unintentional leaks, such as those from mask seals, facial deformities, facial hair, and cuff leaks, can have values for $\lambda$ ranging from about 0.6 to 1.5. A simplified general form for these leaks is similar to that for the intentional leak and is given by the following:

$$Q_{ul} = G_{ul}P^{\lambda\_ul}.$$

The estimate of the leak, $Q_{est}$, is given below where the basis function for the leak is $f$.

$$Q_{est} = gf(P, Qp, Qtot, V, X).$$

In keeping with the generalized formulation for the function $F$ discussed above, a complementary generalized formulation for $f$ is as follows:

$$f = P^{\gamma},$$

where $\gamma$ is the estimate of $\lambda$ in F. For any given $\gamma$, the coefficient g in the function $f$ is found from the following:

$$g = \frac{\overline{Q_{tot}}}{\overline{f}},$$

where the bar denotes an average value over a single breath.

Where $\overline{f} = \overline{P^{\gamma}}$ is assumed, then $\gamma$ can be found using the following methodology. If the average of $P^{\gamma}$, $\overline{P^{\gamma}}$, is sufficiently different between two breathes (A and B), then g and $\gamma$ can be found either directly or iteratively such that:

$$g = \frac{\overline{Q_A}}{P_A^{\gamma}} = \frac{\overline{Q_B}}{P_B^{\gamma}}$$

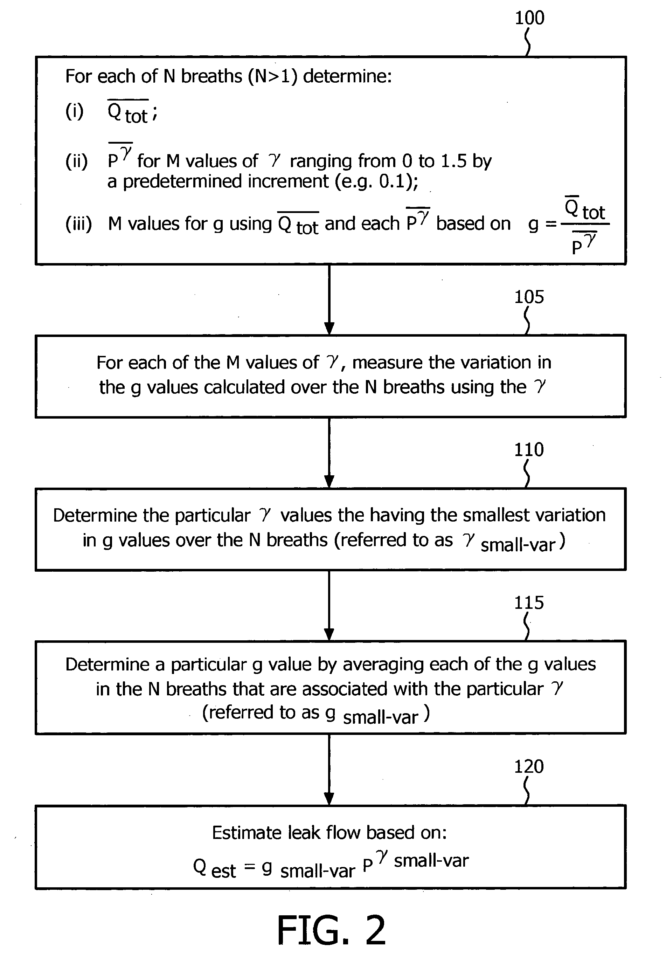

For most leak scenarios, the $G$ and $\lambda$ remain constant for many breaths. During sleep and calm breathing, the variations between breaths are too small to use the above methodology to find g and $\gamma$. Thus, the present invention provides an alternative methodology for fmding g and $\gamma$ that may be used during sleep and clam breathing that employs a statistical method that minimizes the variation of g. FIG. 2 is a flowchart illustrating one exemplary embodiment of such a method. In the exemplary embodiment, the method of FIG. 2 is implemented in controller 64 (i.e., a program or programs implementing the method is stored in the memory portion of controller 64 and is executable by the processing portion of controller 64). In addition, in the exemplary embodiment, after an initial data collection period of a certain number of breaths, the method of FIG. 2 is executed with each new breath so that the leak estimation can be updated with each new breath using the determined g and $\gamma$ values.

Referring to FIG. 2, the method begins at step 100, wherein for each of N breaths (N>1; e.g., 5), the following items are determined: (i) average total flow $\overline{Q_{tot}}$ for the breath; (ii) $\overline{P^\gamma}$ for M different values of $\gamma$ (M>1) ranging from a first predetermined value (e.g., 0) to a second predetermined value (e.g., 1.5) by predetermined increments (e.g., increments of 0.1) (with the result being M different $\overline{P^\gamma}$ values); and (iii) M different values for g using average total flow $\overline{Q_{tot}}$ for the breath and each of the M different $\overline{P^\gamma}$ values for the breath based on

$$g = \frac{\overline{Q_{tot}}}{\overline{f}} \quad \text{where } \overline{f} = \overline{P^\gamma}.$$

In the exemplary embodiment, each of the M different values of $\gamma$ is an integer or a non-integer (positive or negative) real number. Next, at step 105, for each of the M different values of $\gamma$ (e.g., ranging from 0 to 1.5 by 0.1 increments), measure the degree of variation in each of the g values that were calculated over the N breaths in step 100 using that $\gamma$. In the exemplary embodiment, this degree of variation is measured by calculating the standard deviation of the g values. As will appreciated, other methods for determining the degree of variation of the g values may be used, such as, without limitation, the variance, average absolute difference or maximum difference, among others. At step 110, which particular one of values of $\gamma$ that has the g values having the smallest amount of variation (e.g., the smallest standard deviation) associated therewith. That particular one of M values of $\gamma$ shall, for convenience, be refereed to as $\gamma_{small-var}$. Then, at step 115, a particular value, refereed to as $g_{small-var}$ for convenience, is determined by averaging each of the g values in the N breaths that are associated with $\gamma_{small-var}$. Next, at step 120, the leak flow is estimated based on the following:

$$Q_{est} = g_{small-var} P^{\gamma_{small-var}}.$$

In one particular, optional embodiment, once the $\gamma_{small-var}$ is determined as described above, steps 100 to 115 are repeated for the N breaths, using a range of values (e.g., X values) around $\gamma_{small-var}$ with a smaller increment. For example, suppose the first pass search ($\gamma$ = 0 to 1.5 by increments of 0.1) found that $\gamma$ = 0.9 produced a g with the least variation. Then, a new search range of, for example, 0.8 to 1.0 by increments of 0.02 may be used to yield a further refinement of $\gamma$. This iteration process may be repeated any number of times. The new $\gamma_{small-var}$ and $g_{small-var}$ values that are determined after the iterations are complete would then be used in step 120 to determine leak flow.

Furthermore, other iterative methods of searching for $\gamma$ to minimize the variation of g can be employed. One such method would be:

$$\gamma_{k+1} = \gamma_k - \nabla_k,$$

where k is the iteration index and $\nabla_k$ describes the step-size towards minimizing variation in g.

It can be appreciated from the foregoing that the present invention provides an improved leak estimation technique for use in gas delivery systems, such as, without limitation, positive pressure support systems or invasive ventilator systems, particularly in cases, such as when high pressure support levels are employed, where errors in leak estimation will be amplified.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the scope of the appended claims. For example, it is to be understood that the present invention contemplates that, to the extent possible, one or more features of any embodiment can be combined with one or more features of any other embodiment.

**Claims**

1. A method of determining an estimated leak flow $Q_{est}$ in a gas delivery system (50), comprising:

   determining for each of N breaths (N > 1): (i) an average total flow $\overline{Q_{tot}}$ of the gas delivery system, and (ii) M values of $\overline{P^\gamma}$ (M > 1) using M $\gamma$ values, wherein each $P$ is a leak pressure of the gas delivery system and each $\gamma$ value is an integer or non-integer real number;
   determining which one of the $\gamma$ values results in a minimum variation of the values of a coefficient g over the N breaths, wherein for each breath and each $\gamma$ value the coefficient g is determined by $g = \overline{Q_{tot}} / \overline{P^\gamma}$; and
   determining the estimated leak flow $Q_{est}$ using at least the determined one of the $\gamma$ values.

2. The method according to claim 1, wherein the determining the estimated leak flow $Q_{est}$ comprises determining a particular coefficient g using the determined one of the $\gamma$ values, and determining the estimated leak flow $Q_{est}$ using the determined one of the $\gamma$ values and the particular coefficient g.

**3.** The method according to claim 2, wherein the determining a particular coefficient g using the determined one of the $\gamma$ values comprises averaging each of the values of the coefficient $g$ that were determined using the determined one of the $\gamma$ values to provide the particular coefficient g.

**4.** The method according to claim 1, wherein the $\gamma$ values comprises a range of real numbers extending from a first real number to a second real number by predetermined increments.

**5.** The method according to claim 4, wherein the first real number is 0, the second real number is 1.5, and each of the predetermined increments is 0.1.

**6.** The method according to claim 4, wherein the determining the estimated leak flow $Q_{est}$ comprises: (i) determining for each of the N breaths X values of $\overline{P^\gamma}$ (X > 1) using X $\gamma$ values in a first range around the determined one of the $\gamma$ values extending from a third real number to a fourth real number by predetermined second increments, wherein the predetermined second increments are smaller than the predetermined increments, (ii) determining which one of the X $\gamma$ values results in a minimum variation of the values of the coefficient g over the N breaths determined using the X $\gamma$ values, and determining the estimated leak flow $Q_{est}$ using at least the determined one of the X $\gamma$ values.

**7.** The method according to claim 1, wherein the minimum variation is determined based on at least one of standard deviation, variance, average absolute difference or maximum difference.

**8.** A gas delivery system (50), comprising:

a pressure generating system (52, 60) adapted to produce a first flow of gas;
a patient circuit (56, 58) operatively coupled to the pressure generating system; and
a controller (64) operatively coupled to the pressure generating system, the controller being programmed to determine an estimated leak flow $Q_{est}$ in the gas delivery system (50) by:

determining for each of N breaths (N > 1): (i) an average total flow $\overline{Q_{tot}}$ of the gas delivery system, and (ii) M values of $\overline{P^\gamma}$ (M > 1) using M $\gamma$ values, wherein each $P$ is a leak pressure of the gas delivery system and each $\gamma$ value is an integer or non-integer real number;
determining which one of the $\gamma$ values results in a minimum variation of the values of a coefficient g over the N breaths, wherein for each breath and each $\gamma$ value the coefficient $g$ is determined by g = $\overline{Q_{tot}}$ / $\overline{P^\gamma}$ ; and determining the estimated leak flow $Q_{est}$ using at least the determined one of the $\gamma$ values.

**9.** The gas delivery system according to claim 8, wherein the determining the estimated leak flow $Q_{est}$ comprises determining a particular coefficient g using the determined one of the $\gamma$ values, and determining the estimated leak flow $Q_{est}$ using the determined one of the $\gamma$ values and the particular coefficient g.

**10.** The gas delivery system according to claim 9, wherein the determining a particular coefficient g using the determined one of the $\gamma$ values comprises averaging each of the values of the coefficient $g$ that were determined using the determined one of the $\gamma$ values to provide the particular coefficient $g$.

**11.** The gas delivery system according to claim 8, wherein the $\gamma$ values comprises a range of real numbers extending from a first real number to a second real number by predetermined increments.

**12.** The gas delivery system according to claim 11, wherein the first real number is 0, the second real number is 1.5, and each of the predetermined increments is 0.1.

**13.** The gas delivery system according to claim 11, wherein the determining the estimated leak flow $Q_{est}$ comprises: (i) determining for each of the N breaths X values of $\overline{P^\gamma}$ (X > 1) using X $\gamma$ values in a first range around the determined one of the $\gamma$ values extending from a third real number to a fourth real number by predetermined second increments, wherein the predetermined second increments are smaller than the predetermined increments, (ii) determining which one of the X $\gamma$ values results in a minimum variation of the values of the coefficient g over the N breaths determined using the X $\gamma$ values, and determining the estimated leak flow $Q_{est}$ using at least the determined one of the X $\gamma$ values.

**14.** The gas delivery system according to claim 8, wherein the minimum variation is determined based on at least one of standard deviation, variance, average absolute difference or maximum difference.

**Patentansprüche**

**1.** Verfahren zur Ermittlung einer geschätzten Leckströmung *Qest* in einem Gaszufuhrsystem (50), das Folgendes umfasst:

Ermitteln für jeden von N Atemzügen (N > 1): (i) einer durchschnittlichen Gesamtströmung $\overline{Qtot}$ des Gaszufuhrsystems und (ii) von M Werten von $\overline{P^\gamma}$ (M > 1) unter Verwendung von M Werten $\gamma$,

wobei jedes P ein Leckdruck des Gaszufuhrsystems und jeder Wert $\gamma$ eine ganzzahlige oder nicht ganzzahlige reelle Zahl ist,

Ermitteln, welcher der Werte $\gamma$ zu einer Mindeständerung der Werte eines Koeffizienten g während N Atemzügen führt, wobei für jeden Atemzug und jeden Wert $\gamma$ der Koeffizient g er-

mittelt wird durch $g = \dfrac{\overline{Qtot}}{\overline{P^\gamma}}$, und

Ermitteln der geschätzten Leckströmung *Qest* unter Verwendung zumindest des ermittelten Wertes $\gamma$.

2. Verfahren nach Anspruch 1, wobei die Ermittlung der geschätzten Leckströmung *Qest* die Ermittlung eines speziellen Koeffizienten g unter Verwendung des ermittelten Wertes $\gamma$ und die Ermittlung der geschätzten Leckströmung *Qest* unter Verwendung des ermittelten Wertes $\gamma$ und des speziellen Koeffizienten g umfasst.

3. Verfahren nach Anspruch 2, wobei die Ermittlung eines speziellen Koeffizienten g unter Verwendung des ermittelten Wertes $\gamma$ die Mittelwertbildung von jedem der Werte des Koeffizienten g umfasst, die unter Verwendung des ermittelten Wertes $\gamma$ zur Lieferung des speziellen Koeffizienten g ermittelt wurden.

4. Verfahren nach Anspruch 1, wobei die Werte $\gamma$ ein Spektrum an reellen Zahlen umfassen, das sich von einer ersten reellen Zahl bis zu einer zweiten reellen Zahl in vorbestimmten Inkrementen erstreckt.

5. Verfahren nach Anspruch 4, wobei die erste reelle Zahl 0 ist, die zweite reelle Zahl 1,5 ist und jedes der vorbestimmten Inkremente 0,1 ist.

6. Verfahren nach Anspruch 4, wobei die Ermittlung der geschätzten Leckströmung *Qest* Folgendes umfasst: (i) Ermitteln für jeden der N Atemzüge von X Werten von $\overline{P^\gamma}$ (X > 1) unter Verwendung von X Werten $\gamma$ in einem ersten Spektrum um den ermittelten Wert $\gamma$, das sich von einer dritten reellen Zahl bis zu einer vierten reellen Zahl in vorbestimmten zweiten Inkrementen erstreckt, wobei die vorbestimmten zweiten Inkremente kleiner als die vorbestimmten Inkremente sind, ii) Ermitteln, welcher der X Werte $\gamma$ zu einer Mindeständerung der Werte des Koeffizienten g während der unter Verwendung der X Werte $\gamma$ ermittelten N Atemzüge führt, und Ermitteln der geschätzten Leckströmung *Qest* unter Verwendung zumindest des ermittelten der X Werte $\gamma$.

7. Verfahren nach Anspruch 1, wobei die Mindeständerung anhand von zumindest entweder Standardabweichung, Varianz, durchschnittlicher absoluter Differenz oder maximaler Differenz ermittelt wird.

8. Gaszufuhrsystem (50), das Folgendes umfasst:

ein Druckerzeugungssystem (52, 60), das für die Erzeugung einer ersten Gasströmung ausgelegt ist,
einen Patientenkreislauf (56, 58), der in Wirkbeziehung mit dem Druckerzeugungssystem gekoppelt ist, und
einen Controller (64), der in Wirkbeziehung mit dem Druckerzeugungssystem gekoppelt ist, wobei der Controller so programmiert ist, dass er eine geschätzten Leckströmung *Qest* in einem Gaszufuhrsystem (50) ermittelt durch:

Ermitteln für jeden von N Atemzügen (N > 1): (i) einer durchschnittlichen Gesamtströmung $\overline{Qtot}$ des Gaszufuhrsystems und (ii) von M Werten von $\overline{P^\gamma}$ (M > 1) unter Verwendung von M Werten $\gamma$,
wobei jedes P ein Leckdruck des Gaszufuhrsystems und jeder Wert $\gamma$ eine ganzzahlige oder nicht ganzzahlige reelle Zahl ist,
Ermitteln, welcher der Werte $\gamma$ zu einer Mindeständerung der Werte eines Koeffizienten g während N Atemzügen führt, wobei für jeden Atemzug und jeden Wert $\gamma$ der Koeffizient g ermittelt wird durch

$g = \dfrac{\overline{Qtot}}{\overline{P^\gamma}}$ und

Ermitteln der geschätzten Leckströmung *Qest* unter Verwendung zumindest des ermittelten Wertes $\gamma$.

9. Gaszufuhrsystem nach Anspruch 8, wobei die Ermittlung der geschätzten Leckströmung *Qest* die Ermittlung eines speziellen Koeffizienten g unter Verwendung des ermittelten Wertes $\gamma$ und die Ermittlung der geschätzten Leckströmung *Qest* unter Verwendung des ermittelten Wertes $\gamma$ und des speziellen Koeffizienten g umfasst.

10. Gaszufuhrsystem nach Anspruch 9, wobei die Ermittlung eines speziellen Koeffizienten g unter Verwendung des ermittelten Wertes $\gamma$ die Mittelwertbildung von jedem der Werte des Koeffizienten g umfasst, die unter Verwendung des ermittelten Wertes $\gamma$ zur Lieferung des speziellen Koeffizienten g ermittelt wurden.

**11.** Gaszufuhrsystem nach Anspruch 8, wobei die Werte γ ein Spektrum an reellen Zahlen umfassen, das sich von einer ersten reellen Zahl bis zu einer zweiten reellen Zahl in vorbestimmten Inkrementen erstreckt.

**12.** Gaszufuhrsystem nach Anspruch 11, wobei die erste reelle Zahl 0 ist, die zweite reelle Zahl 1,5 ist und jedes der vorbestimmten Inkremente 0,1 ist.

**13.** Gaszufuhrsystem nach Anspruch 11, wobei die Ermittlung der geschätzten Leckströmung $Q_{est}$ Folgendes umfasst: (i) Ermitteln für jeden der N Atemzüge von X Werten von $\overline{P^\gamma}$ (X > 1) unter Verwendung von X Werten von γ in einem ersten Spektrum um den ermittelten Wert γ, das sich von einer dritten reellen Zahl bis zu einer vierten reellen Zahl in vorbestimmten zweiten Inkrementen erstreckt, wobei die vorbestimmten zweiten Inkremente kleiner als die vorbestimmten Inkremente sind, (ii) Ermitteln, welcher der X Werte γ zu einer Mindeständerung der Werte des Koeffizienten g während der unter Verwendung der X Werte γ ermittelten N Atemzüge führt, und Ermitteln der geschätzten Leckströmung $Q_{est}$ unter Verwendung zumindest des ermittelten der X Werte γ.

**14.** Gaszufuhrsystem nach Anspruch 8, wobei die Mindeständerung anhand von zumindest entweder Standardabweichung, Varianz, durchschnittlicher absoluter Differenz oder maximaler Differenz ermittelt wird.

**Revendications**

**1.** Procédé de détermination d'un débit de fuite estimé $Q_{est}$ dans un système d'alimentation de gaz (50) comprenant :

la détermination, pour chacune des N respirations (N > 1) : (1) d'un débit total moyen $\overline{Q_{tot}}$ du système d'alimentation de gaz et (2) des M valeurs de $\overline{P^\gamma}$ (M > 1) en utilisant les M valeurs γ, dans lequel chaque P est une pression de fuite du système d'alimentation de gaz et chaque valeur γ est un nombre réel entier ou un nombre réel non entier ;
la détermination de celle des valeurs γ qui engendre une variation minimale des valeurs d'un coefficient g sur les N respirations, dans lequel, pour chaque respiration et pour chaque valeur γ, le coefficient g est déterminé par

$$g = \frac{\overline{Qtot}}{\overline{P^\gamma}} \; ; \text{et}$$

la détermination du débit de fuite estimé $Q_{est}$ en

utilisant celle déterminée des valeurs γ.

**2.** Procédé selon la revendication 1, dans lequel la détermination du débit de fuite estimé $Q_{est}$ comprend la détermination d'un coefficient g particulier en utilisant celle déterminée des valeurs γ et la détermination du débit de fuite estimé $Q_{est}$ en utilisant celle déterminée des valeurs γ et le coefficient g particulier.

**3.** Procédé selon la revendication 2, dans lequel la détermination d'un coefficient g particulier en utilisant celle déterminée des valeurs γ comprend le calcul de la moyenne de chacune des valeurs du coefficient g qui ont été déterminées en utilisant celle déterminée des valeurs γ pour fournir le coefficient g particulier.

**4.** Procédé selon la revendication 1, dans lequel les valeurs γ comprennent une plage de nombres réels s'étendant d'un premier nombre réel à un deuxième nombre réel par incréments prédéterminés.

**5.** Procédé selon la revendication 4, dans lequel le premier nombre réel est 0, le deuxième nombre réel est 1,5 et chacun des incréments prédéterminés est 0,1.

**6.** Procédé selon la revendication 4, dans lequel la détermination du débit de fuite estimé $Q_{est}$ comprend :
(1) la détermination, pour chacune des N respirations, de X valeurs de $\overline{P^\gamma}$ (X > 1) en utilisant X valeurs γ dans une première plage autour de l'une déterminée des valeurs γ s'étendant d'un troisième nombre réel à un quatrième nombre réel par des deuxièmes incréments prédéterminés, dans lequel les deuxièmes incréments prédéterminés sont plus petits que les incréments prédéterminés, (2) la détermination de celle des X valeurs γ qui engendre une variation minimale des valeurs du coefficient g sur les N respirations déterminées en utilisant les X valeurs γ et la détermination du débit de fuite estimé $Q_{est}$ en utilisant au moins celle déterminée des X valeurs γ.

**7.** Procédé selon la revendication 1, dans lequel la variation minimale est déterminée sur la base d'au moins l'un d'un écart-type, d'une variance, d'une différence absolue moyenne ou d'une différence maximale.

**8.** Système d'alimentation de gaz (50) comprenant :

un générateur de pression (52, 60) apte à produire un premier débit de gaz ;
un circuit de patient (56, 58) couplé de manière opérationnelle au générateur de pression ; et
un organe de contrôle (64) couplé de manière opérationnelle au générateur de pression, l'organe de contrôle étant programmé pour déterminer un débit de fuite estimé $Q_{est}$ dans le système d'alimentation de gaz (50) par :

la détermination, pour chacune des N respirations (N > 1) : (1) d'un débit total moyen $\overline{Q_{tot}}$ du système d'alimentation de gaz et (2) de M valeurs de $\overline{P^\gamma}$ (M > 1) en utilisant M valeurs $\gamma$, dans lequel chaque $P$ est une pression de fuite du système d'alimentation de gaz et chaque valeur $\gamma$ est un nombre réel entier ou un nombre réel non entier ;
la détermination de celle des valeurs $\gamma$ qui engendre une variation minimale des valeurs d'un coefficient g sur les N respirations, dans lequel, pour chaque respiration et pour chaque valeur $\gamma$, le coefficient $g$ est

déterminé par $g = \dfrac{\overline{Qtot}}{\overline{P^\gamma}}$ ; et

la détermination du débit de fuite estimé $Q_{est}$ en utilisant celle déterminée des valeurs $\gamma$.

9. Système d'alimentation de gaz selon la revendication 8, dans lequel la détermination du débit de fuite estimé $Q_{est}$ comprend la détermination d'un coefficient g particulier en utilisant celle déterminée des valeurs $\gamma$ et la détermination du débit de fuite estimé $Q_{est}$ en utilisant celle déterminée des valeurs $\gamma$ et le coefficient g particulier.

10. Système d'alimentation de gaz selon la revendication 9, dans lequel la détermination d'un coefficient g particulier en utilisant celle déterminée des valeurs $\gamma$ comprend le calcul de la moyenne de chacune des valeurs du coefficient g qui ont été déterminées en utilisant celle déterminée des valeurs $\gamma$ pour fournir le coefficient g particulier.

11. Système d'alimentation de gaz selon la revendication 8, dans lequel les valeurs $\gamma$ comprennent une plage de nombres réels s'étendant d'un premier nombre réel à un deuxième nombre réel par incréments prédéterminés.

12. Système d'alimentation de gaz selon la revendication 11, dans lequel le premier nombre réel est 0, le deuxième nombre réel est 1,5 et chacun des incréments prédéterminés est 0,1.

13. Système d'alimentation de gaz selon la revendication 11, dans lequel la détermination du débit de fuite estimé $Q_{est}$ comprend : (1) la détermination, pour chacune des N respirations, de X valeurs de $\overline{P^\gamma}$ (X > 1) en utilisant X valeurs $\gamma$ dans une première plage autour de l'une déterminée des valeurs $\gamma$ s'étendant d'un troisième nombre réel à un quatrième nombre réel par des deuxièmes incréments prédéterminés, dans lequel les deuxièmes incréments prédéterminés sont plus petits que les incréments prédéterminés, (2) la détermination de celle des X valeurs $\gamma$ qui engendre une variation minimale des valeurs du coefficient g sur les N respirations déterminées en utilisant les X valeurs $\gamma$ et la détermination du débit de fuite estimé $Q_{est}$ en utilisant au moins celle déterminée des X valeurs $\gamma$.

14. Système d'alimentation de gaz selon la revendication 8, dans lequel la variation minimale est déterminée sur la base d'au moins l'un d'un écart-type, d'une variance, d'une différence absolue moyenne ou d'une différence maximale.

FIG. 1

100

For each of N breaths (N>1) determine:

(i)   $\overline{Q_{tot}}$ ;

(ii)  $\overline{P^{\gamma}}$ for M values of $\gamma$ ranging from 0 to 1.5 by
      a predetermined increment (e.g. 0.1);

(iii) M values for g using $\overline{Q_{tot}}$ and each $\overline{P^{\gamma}}$ based on $g = \dfrac{\overline{Q_{tot}}}{\overline{P^{\gamma}}}$

105

For each of the M values of $\gamma$, measure the variation in
the g values calculated over the N breaths using the $\gamma$

110

Determine the particular $\gamma$ values the having the smallest variation
in g values over the N breaths (referred to as $\gamma_{small\text{-}var}$)

115

Determine a particular g value by averaging each of the g values
in the N breaths that are associated with the particular $\gamma$
(referred to as $g_{small\text{-}var}$)

120

Estimate leak flow based on:

$Q_{est} = g_{small\text{-}var} \, P^{\gamma \, small\text{-}var}$

# FIG. 2

**REFERENCES CITED IN THE DESCRIPTION**

**Patent documents cited in the description**

- US 2005224078 A

- WO 2010044038 A